# EUROPEAN PATENT APPLICATION

(11) **EP 4 789 679 A1**
(43) Date of publication of application: **12.08.2026**
(21) Application number: 25157239.2
(22) Date of filing: 11.02.2025
(51) Int. Cl.: A61K 38/07, A61P 11/00, A61P 29/00

(54) **KEDG PEPTIDE FOR TREATING RESPIRATORY DISEASES**

(71) Applicant: Capsa Sanigant GmbH, 7000 Chur (CH)
(72) Inventor: DUDKOV, Alexander, 1204 GENEVA (CH); HUBER, Daniel, 1248 HERMANCE (CH)
(74) Representative: Santarelli

(57) **Abstract**

The present invention relates to the field of therapeutic peptides for use in the treatment or the prevention of respiratory diseases, particularly acute respiratory distress syndrome. The invention provides a peptide consisting of the amino acid sequence of KEDG, as well as therapeutic uses of such peptide.

## Description

### FIELD OF THE INVENTION

The present invention relates to the field of therapeutic peptides for use in the treatment or the prevention of respiratory disease, particularly acute respiratory distress syndrome.

### BACKGROUND OF THE INVENTION

Acute respiratory distress syndrome (ARDS) is the most severe clinical manifestation of acute lung injury syndrome, a common cause of which is pneumonia of various etiologies. ARDS is characterized by diffuse infiltration and violation of the alveolar-capillary barrier due to death or dysfunction of the alveolar cells of the pulmonary capillaries, which leads to severe hypoxemia. In many cases, severe hypoxemia requires life-sustaining mechanical ventilation and the delivery of high concentrations of supplemental oxygen. ARDS often leads to death (from 30% to 60%). The main approach to treatment includes ventilation assistance and support of the cardiovascular system, followed by the use of glucocorticosteroids and surfactant replacement therapy.

Currently, there are few effective drugs for the treatment of acute lung injury. At the same time, they all have pronounced side effects. The use of therapeutic peptides could be an effective and safe method of prevention and correction of many pathological conditions. In this regard, the inventors have surprisingly found that KEDG peptide is useful in the prevention and treatment of ARDS.

### DETAILED DESCRIPTION

According to a first aspect, the present invention pertains to a peptide consisting of the amino acid sequence of KEDG (SEQ ID NO: 1) for use in the treatment or prevention of acute respiratory disease syndrome in a mammal.

The peptide is an ultrashort peptide, such as an isolated or synthesized ultrashort peptide. As used herein, the term "ultrashort peptide" means a peptide consisting in only two to seven amino acids, particularly a peptide consisting in two, three or four amino acids. The term "peptide" as used herein also includes pharmaceutically acceptable salt of said peptide.

The peptide, as described herein, may be provided in an isolated or purified form. An isolated form refers to a state in which the peptide is substantially separated from other components that are naturally associated with it, such as cellular components, other peptides, or proteins. A purified form refers to a higher level of separation, where the peptide has a purity of preferably at least 95%, more preferably at least 98%, or higher. These forms facilitate the use of the peptide in pharmaceutical compositions for the treatment or prevention of accute respiratory disease syndrome in a mammal.

The peptide may be synthetized by any suitable methods such as chemical methods (e.g. in solid phase or in liquid phase), chemoenzymatic methods or biosynthesis. Alternatively, the peptide may be obtained from natural products by extraction.

The peptide consists in the amino acid sequence of **KEDG** (SEQ ID NO: 1), or a pharmaceutically acceptable salt thereof . In the present specification, amino acid sequences are written from N-terminus to C-terminus unless otherwise stated. Thus, it is intended by "KEDG" the following peptide : H-Lys-Glu-Asp-Gly-OH (SEQ ID NO: 1).

Preferably, the peptide comprises L-amino acids, preferably consists in L-amino acids. Thus, the peptide is preferably L-Lys-L-Glu-L-Asp-L-Gly (SEQ ID NO: 1)

More preferebly, the peptide is a tetrapeptide having an amino acid sequence of KEDG (SEQ ID NO: 1), wherein the amino acids are L-amino acids, and wherein the amino acid sequence is written from N-terminus to C-terminus.

As used herein, the term "pharmaceutically acceptable salt" refers to a salt form of the peptide that is suitable for pharmaceutical use. This salt may be formed by the reaction of the peptide with appropriate acids or basis. Non limitative example of pharmaceutically acceptable salts include acetate, hydrochloride, sulfate, citrate and phosphate salts.

As used herein, the terms "treatment" or "treating" and the like generally mean obtaining a desired pharmacological and physiological effect, leading to the inhibition and/or regretion of a disease. The effect may be therapeutic in terms of a partial or complete cure of a disease, condition, symptom or adverse effect attributed to the disease.

As used herein, the terms "prevention" or "preventing" and the like generally mean obtaining a prophylactic effect leading to the prevention or partial prevention of a disease, symptom or condition thereof. These terms can notably cover preventing the disease from occurring in a subject which may be predisposed to the disease but has not yet been diagnosed as having it such as a preventive early asymptomatic intervention.

As used herein, acute respiratory disease syndrome or ARDS means a severe form of respiratory failure that may be characterized by:
- respiratory distress,
- hypoxemia,
- diffuse and bilateral lung injury (that may be indentified by thoracic radiography), and/or
- an inflammation in the lungs.

ARDS may be induced by various causes such as pneumopathy, pulmonary embolism, polytraumatism, eclampsia, sepsis, etc.

ARDS may be defined more precisely by Berlin criteria which provide the following criteria.
(1) Acute onset: ARDS must occur within 7 days of an identifiable trigger (such as infection, trauma, etc);
(2) Hypoxemia: Hypoxemia is measured using the PaO₂/FiO₂ ratio (FiO₂ refers to the fraction of inspired oxygen, and PaO₂ refers to the partial pressure of oxygen in the blood)
   - Mild ARDS: PaO₂/FiO₂ ratio between 200 and 300 mmHg ;
   - Moderate ARDS: PaO₂/FiO₂ ratio between 100 and 200 mmHg;
   - Severe ARDS: PaO₂/FiO₂ ratio less than 100 mmHg.
(3) Non-cardiac pulmonary edema: ARDS is caused by inflammation in the lungs, not by heart failure. This means that pulmonary edema must be non-cardiogenic (not related to heart failure or volume overload).
(4) Bilateral infiltrates on chest imaging : Chest X-rays or CT scans show infiltrates affecting both lungs, which is characteristic of ARDS.

In the present application, unless otherwise indicated, all ranges of values are to be understood as including the limits.

ARDS may comprise several grades. A first grade, called "exsudative", may be characterized by extravasation of fluids and proteins into the pulmonary alveolus. A second grade, called "proliferative", may be characterized by the recruitment of macrophages in lung and often excessive secretion of cytokines (cytokine storm). A third grade, called "healing" grade, may be complicated by the formation of lung fibrosis.

As mentioned above, the peptide is used in the treatment or prevention in a mammal.

According to a first embodiment, the peptide may be administered to a mammal that suffer from ARDS. The peptide may be administered at an early stage of the disease, preferably at the "proliferative" grade. The peptide may also be administered at a later stage, preferably at the "healing" grade.

According to a second embodiment, the peptide may be administered to a mammal which does not suffer from ARDS, for the sake of preventing ARDS (prophylaxie).

As used herein, the term "mammal" may refer to a human or an animal.

Thus, in a first embodiment, the peptide for use according to the invention is administered to a human.

Advantageously, according to this first embodiment, the peptide for use according to the invention, wherein said peptide is administered at a dose from 0.2 µg/kg to 150 µg/kg of body weight per day.

Preferably, the peptide may be administered to a human at a dose from 0.2 µg/kg to 20 µg/kg per day, more preferably at a dose from 2 µg/kg to 20 µg/kg per day.

According to a second embodiment, the peptide for use according to the invention is administered to an animal.

According to this second embodiment, the animal may be a pet or a breeding animal.

Non limitative examples of pets are dogs, cats, rabbits, guinea pigs, hamsters, ferrets, gerbils, mice, or chinchillas, preferably dogs or cats. Non limitative examples of breeding animals are horses, donkeys, cows, pigs, sheep, or goats.

Preferably, the animal to be treated with the peptide is a pet, such as a cat or a dog.

Advantageously, the peptide for use according to the invention is administered at a dose from 0.2 µg/kg to 20 µg/kg per day.

Preferably, the peptide may be administered to an animal, for example a pet, preferably a cat or a dog, at a dose from 0.2 µg/kg to 20 µg/kg per day.

The peptide may be administered to the mammal through various routes of administration, that may be local or systemic. Non limitative examples of routes of administrations are intravenous, subcutaneous or intramuscular injections, oral administration, intranasal administration or pulmonary administration.

In a first preferred route of administration, the peptide for use according to the invention is delivered by intranasal administration.

In this case, the peptide may be administered as a spray or as a solution through the nose. Such route of administration allows a fast absorption by the nasal mucosa. This route of administration is particularly advantageous when the peptide of the invention is used for preventing ARDS.

According to the first embodiment, the peptide may be administered to a human by intranasal administration, at a dose from 0.2 µg/kg to 150 µg/kg of body weight per day, preferably at a dose from 0.2 µg/kg to 20 µg/kg per day, still more preferably at a dose from 2 µg/kg to 20 µg/kg per day. Such dosage and route of administration are particularly advantageous for preventing ARDS. Preferably, the peptide is administered during 10 days according to this regimen.

According to the second embodiment, the peptide may be administered to a pet by intranasal route, at a dose from 0.2 µg/kg to 20 µg/kg of body weight per day. Such dosage and route of administration are particularly advantageous for preventing ARDS. Preferably, the peptide is administered during 10 days according to this regimen.

In a second preferred route of administration, the peptide for use according to the invention is delivered by intramuscular administration.

In this case, the peptide is injected directly into a muscle. Typical injection sites include the deltoid, vastus lateralis (thigh), and gluteal muscles.

According to the first embodiment, the peptide may be administered to a human by intramuscular route, at a dose from 0.2 µg/kg to 150 µg/kg of body weight per day, preferably at a dose from 0.2 µg/kg to 20 µg/kg per day, still more preferably at a dose from 2 µg/kg to 20 µg/kg per day. Such dosage and route of administration are particularly advantageous for treating ARDS. Preferably, the peptide is administered during 10 days according to this regimen.

According to the second embodiment, the peptide may be administered to a pet by intramuscular route, at a dose from 0.2 µg/kg to 20 µg/kg of body weight per day. Such dosage and route of administration are particularly advantageous for treating ARDS. Preferably, the peptide is administered during 10 days according to this regimen.

Alternatively, the peptide may administered by inhalatory route. In this case, the peptide may be delivered through an aerosol administered by the nose or mouth of the mammal. Such route of administration allows a direct delivery to the lung and rapid absorption via the lung epithelium.

The peptide can also be administered orally, such as locally on the buccal / throat mucosa. In this case, the peptide may be administered as a spray or as a solution by the mouth. Such route of administration allows a direct delivery to the throat mucosa and the pharynx.

The peptide may be administered together with other drugs or active principles. Preferably, said other drugs or active principles may be useful for treating any respiratory diseases. Such drugs useful for treating any respiratory diseases may have a bronchodilatotor effect, an analgesic effect, and/or an anti-inflammatory effect. Non limitative examples of such drugs or active principles useful for treating respiratory diseases are corticosteroids, bronchodilatotors, phophodiesterase-4 inhibitors, mucolytics, expectorants, antibiotics, immunosuppressive agents, antiviral agents, monoclonal antibodies, and Beta-2 receptors agonists.

Non limitative examples of bronchodilatotors are Salbutamol, Formotérol, Tiotropium and Ipratropium.

A non limitative example of phosphodiesterase-4 (PDE-4) inhibitors is Roflumilast.

Non limitative examples of mucolytics and expectorants are Acetylcysteine and Carbocysteine

Non limitative examples of antibiotics are Amoxicillin, Cephalosporins and Azithromycin.

Non limitative examples of immunosuppressive agents are Azathioprine, Methotrexate and Cyclophosphamide.

Non limitative examples of antiviral agents are Oseltamivir (Tamiflu) and Remdesivir.

Non limitative examples of monoclonal antibodies are Omalizumab and Dupilumab.

Non limitative examples of beta-2 agonists are Salmeterol and Formoterol.

Preferably, the peptide for use according to the invention is administered together with corticosteroids.

Non limitative examples of corticosteroids are Prednisone, Méthylprednisolone, Budesonide and Fluticasone.

According to a second aspect of the invention, provided is a pharmaceutical composition comprising a peptide consisting of the amino acid sequence of KEDG (SEQ ID NO: 1), or an acceptable salt thereof, for use in the treatment or prevention of acute respiratory disease syndrome in a mammal.

Preferably, the peptide in the pharmaceutical composition is present at effective dosage, either therapeutically effective or prophylactically effective.

Advantageously, the pharmaceutical composition for use according to the invention comprises the peptide consisting of the amino acid sequence of KEDG (SEQ ID NO: 1) at a concentration from 50 µg/ml to 500 µg/ml .

Preferably, the pharmaceutical composition according to the invention comprises the peptide at a concentration from 50 µg/ml to 500 µg/ml.

The pharmaceutical composition for use according to the invention may further comprises a pharmaceutically acceptable carrier.

The pharmaceutical composition for use according to the invention may comprise the peptide as disclosed herein, and one or more other drugs or active principles, as disclosed above.

Pharmaceutically acceptable carriers are well known in the art. Non limitative examples of pharmaceutically acceptable carriers are aqueous solutions such as (sterile) water, physiological solution, saline solution for injection, physiologically buffered saline, other solvents or vehicles (glycols, glycerol, oils or injectable organic esters, alcohol, fats, waxes), and inert solids.

Preferably, the pharmaceutically acceptable carrier is physiological or saline solution.

The pharmaceutically acceptable carrier may further contain physiologically acceptable compounds that act for example to stabilize or to increase the absorption of the peptide. Such physiologically acceptable compounds include, for example, carbohydrates, such as glucose, sucrose ordextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the route of administration of the composition. Pharmaceutically acceptable adjuvants, buffering agents, dispersing agents, and the like, may also be incorporated into the pharmaceutical compositions.

The peptide may also be formulated in the pharmaceutical composition according to the invention in liposomes.

According to another aspect of the invention, provided is a product comprising a peptide consisting of the amino acid sequence of KEDG (SEQ ID NO: 1), or a pharmaceutically acceptable salt thereof, and a corticosteroid, as a combination product for simultaneous, separate or sequential use in the treatment or the prevention of acute respiratory disease syndrome in a mammal.

Such a product is a kit-of-parts comprising KEDG peptide as described above, including advantageous and preferential features.

The present invention also concerns a method for the prophylactic and/or therapeutic treatment of ARDS in a mammal, comprising administering to the mammal a peptide consisting of the amino acid sequence of KEDG (SEQ ID NO: 1).

All the preferential features and embodiments described above applies to the present method.

The present invention also concerns a use of a peptide consisting of the amino acid sequence of KEDG (SEQ ID NO: 1) for the manufacture of a medicament for treating or preventing ARDS in a mammal.

All the preferential features and embodiments described above applies to the present use.

### BRIEF DESCRIPTION OF THE DRAWINGS

Figure 1 represents the effect of KEDG peptide at a dose of 100 µg/ml on cell viability (%) of Wi-38 cells (human lung fibroblasts).
Figure 2 represents the effect of KEDG peptide on cell viability (%) of V79 cells (Chinese hamster lung cells).
Figure 3 shows the effect of KEDG and AEDL peptide on the weight of rats at day 1 and 3 of the treatment.
Figure 4 shows the effect of KEDG and AEDL peptide on the shortness of breath score of rats at day 2 and 3 of the treatment.

### EXAMPLES

The following examples are provided for the sake of illustrations, and does not limit the scope of the invention.

### Example 1: In vitro study of bronchoprotective effect of KEDG peptide

The effect of KEDG peptide on the viability of Wi-38 (human lung fibroblasts) cell lines and V79 (cells of Chinese hamster lungs) have been investigated in order to establish its bronchoprotective effect. Wi-38 and V79 cells were cultivated in a culture medium with fetal bovine serum (FBS) and without FBS. Cultivation cells with FBS is a model of normal condition of cell grow, which is used as positive control in this experiment. Cultivation of cells without FBS is a model of nutrients restriction and stress, which is used as negative control in the experiment (stress model), without adding KEDG peptide. KEDG peptide was added in the culture medium to reach a concentration of 100 µg/ml (on the basis of the culture medium volume). After 48 h of incubation, cell viability was assessed using MTS test [Buranaamnuay K. The MTT assay application to measure the viability of spermatozoa: A variety of the assay protocols. Open Vet J. 2021; 11(2):251-269. doi: 10.5455/OVJ.2021.v11.i2.9].

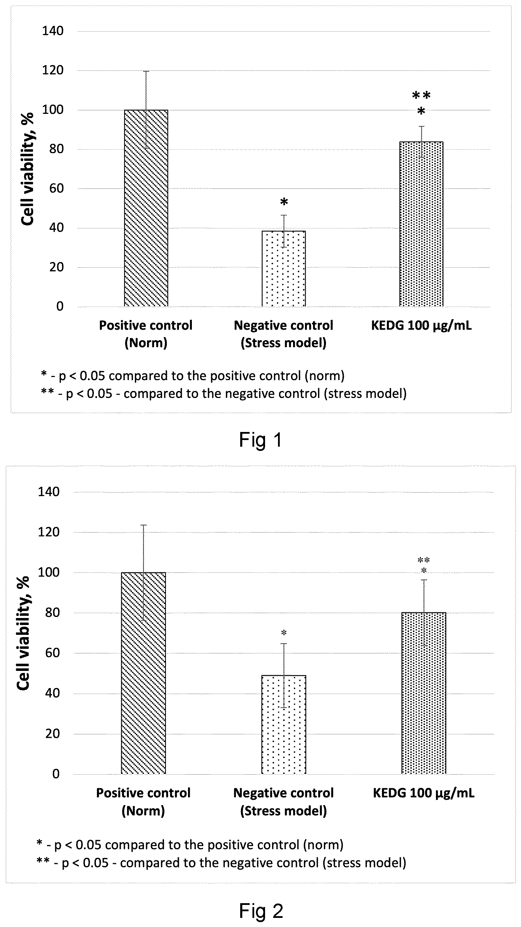

Results for cell viability of Wi-38 cell line after 48h of incubation with KEDG peptide are provided in the Figure 1. KEDG peptide at a dose of 100 µg/ml increased the viability of Wi-38 cells by 40% after 48h of incubation, compared to the negative control (stress model).

Results for cell viability of V79 cell line after 48h of incubation with KEDG peptide are provided in the Figure 2. KEDG peptide at a dose of 100 µg/ml increased the viability of V79 cells by 260 % (2,6 times) after 48h of incubation, compared to to the negative control (stress model).

Such results demonstrate the protective effect on pulmonary cells of KEDG peptide on both human and animal (rodent) pulmonary cell lines, as well as the absence of cytotoxicity towards healthy pulmonary cells. KEDG peptide prevents pulmonary cells death in stress condition. Stress condition is one of the most important factor of ARDS and other lung pathology processes. Thus these data show that KEDG peptide can prevent ARDS pathology processes in lung tissue of human and rodents.

### Example 2: In vivo study of KEDG peptide on rodent model of ARDS

The pharmacological activity of KEDG peptide was assessed in vivo on an rodent model of acute respiratory distress syndrome (ARDS). ARDS was induced by a single endotracheal injection of lipopolysaccharides (LPS) from Escherichia coli in male Wistar rats. There are many approaches for ARDS modeling in animals using various chemical inducers, among which LPS of the bacterial wall of gram-negative bacteria is most widely used. ARDS modeling by endotracheal injection of LPS into the cell wall of gram-negative bacteria has a high similarity with human clinical pathology. In the lungs of animals, the migration of neutrophils and monocytes increases under the action of LPS due to the complex interaction of cytokines, chemokines and adhesion molecules. This model is well reproducible and allows to obtain the characteristic signs of a "cytokine storm" for ARDS. AEDL peptide, which is a bronchoprotector of reference, was used as a positive control. Peptides were administered to rats by intranasal injection at a dose of 0.2 µg/kg, once a day for 3 days, starting one hour after LPS administration. Experiments were conducted on several experimental groups, each comprising 8 animals. A first experimental group was a negative control (ARDS induced, no administration of peptides), a second experimental group received KEDG peptide at 0.2 µg/kg after induction of ARDS, and a third experimental group received AEDL peptide at 0.2 µg/kg after induction of ARDS (positive control). The effectiveness of peptides was assessed by monitoring the evolution of body weight (considering that ARDS induces a significant decrease in body weight), and by evaluating the shortness of breath (that is a symptoma of ARDS). Shortness of breath is depicted through a score from 0 to 8, corresponding to the number of animals suffering from a severe shortness of breath within each group. Shortness of breath is one of the main signs of ARDS. Three signs of shortness of breath were observed in rats in the experiment. First, when the rat is breathing, it strongly retracts its sides, and then sharply inflates them, as if trying to help itself breathe with its stomach. Second, blueness of ears, paws, mucous membranes, claws. Normally, they have a pink color, and with shortness of breath induced by ARDS they acquire a purple hue. Third, opening the mouth when breathing. Normally, rats do not breathe through their mouths. When the animal experiences an attack of suffocation, it begins to open its mouth to inhale more air.

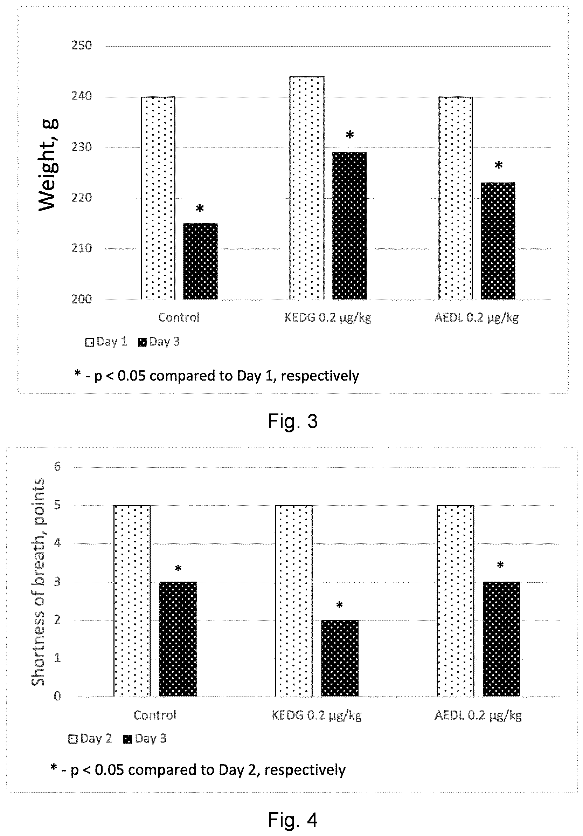

Figure 3 shows the evolution of body weight between day 1 (after administration of LPS) and day 3. In control group (ARDS without treatment) a statistically significant decrease by 8-11% (9.5 ± 1.5%, M ± m) in body weight was revealed between day 1 and day 3 of the experiment. M is the arithmetic mean, and m is the error of the arithmetic mean. In the group with applying KEDG peptide the decrease of body weight was 6.0 ± 0.5%. In the group with applying AEDL peptide the decrease of body weight was 7.5 ± 0.5%. According to these data, KEDG peptide is more effective than AEDL peptide in limiting the loss of weight induced by ARDS.

Figure 4 shows the evolution of shortness of breath score between day 2 and day 3 of the experiment. On day 2, the number of animal suffering from severe shortness of breath was high for each group (5). On day 3, the number of animal suffering from severe shortness of breath decreased from 5 to 3 for the negative control group (self recovery), from 5 to 2 for the group receiving KEDG peptide, and from 5 to 3 for the group receiving AEDL peptide. According to these data, KEDG peptide was more effective than AEDL peptide (bronchoprotector of reference) for reducing the severity of shortness of breath in ARDS rats.

As a conclusion, ARDS induces a significant lose of weight and severe shortness of breath in rats. Daily intranasal administration of a dose of 0.2 µg/kg of KEDG peptide induces a limited lose of weight, and a reduction of shortness of breath in rats compared to control, indicating an improvement in the well-being of rats suffering from ARDS. The bronchoprotector effect of KEDG peptide (its protective effect on pulmonary cell) and its efficiency for the treatment of ARDS is therefore demonstrated *in vivo* on a ARDS rat model.

## Claims

1. A peptide consisting of the amino acid sequence of KEDG (SEQ ID NO: 1) for use in the treatment or prevention of acute respiratory disease syndrome in a mammal.

2. The peptide for use of claim 1, wherein said peptide is administered to a human.

3. The peptide for use of claim 2, wherein said peptide is administered at a dose from 0.2 µg/kg to 150 µg/kg of body weight per day.

4. The peptide for use of claim 1, wherein said peptide is administered to an animal.

5. The peptide for use of claim 4, wherein said peptide is administered at a dose from 0.2 µg/kg to 20 µg/kg per day .

6. The peptide for use of of any one of the claims 1-5, wherein the peptide is delivered by intranasal administration.

7. The peptide for use of any one of the claims 1-5, wherein said peptide is delivered by intramuscular administration.

8. The peptide for use of any one of the above claims, wherein said peptide is administered together with corticosteroids.

9. A pharmaceutical composition comprising a peptide consisting of the amino acid sequence of KEDG (SEQ ID NO: 1), or an acceptable salt thereof, for use in the treatment or prevention of acute respiratory disease syndrome in a mammal.

10. The pharmaceutical composition for use of claim 9, wherein said pharmaceutical composition comprises the peptide consisting of the amino acid sequence of KEDG (SEQ ID NO: 1) at a concentration from 50 µg/ml to 500 µg/ml .

11. The pharmaceutical composition for use according to any one of claims 9 or 10, wherein said composition further comprises a pharmaceutically acceptable carrier.

12. A product comprising a peptide consisting of the amino acid sequence of KEDG (SEQ ID NO: 1), or a pharmaceutically acceptable salt thereof, and a corticosteroid, as a combination product for simultaneous, separate or sequential use in the treatment or the prevention of acute respiratory disease syndrome in a mammal.
